# EUROPÄISCHE PATENTANMELDUNG

(11) **EP 2 848 626 A2**
(43) Veröffentlichungstag der Anmeldung: **18.03.2015**
(21) Anmeldenummer: 14179696.1
(22) Anmeldetag: 04.08.2014
(51) Int. Cl.: C07K 1/34, B01J 19/24, B01D 61/00

(54) **Verfahren zur Herstellung eines Produktes mittels Dialyse**

(30) Priorität: 05.08.2013 DE 102013215380; 11.09.2013 DE 102013218239
(71) Anmelder: TETEC Tissue Engineering Technologies AG, 72770 Reutlingen (DE)
(72) Erfinder: Mollenhauer, Jürgen, 72764 Reutlingen (DE); Joseph, Heinz Walter, 79108 Freiburg (DE); Wurst, Helmut, 72770 Reutlingen (DE)
(74) Vertreter: Patentanwälte Ruff, Wilhelm, Beier, Dauster & Partner

(57) **Zusammenfassung**

Die Erfindung betrifft ein Verfahren zur Herstellung eines Produktes, vorzugsweise in Form einer funktionalisierten hochmolekularen Komponente wie beispielsweise eines funktionalisierten Proteins, umfassend die folgenden Schritte:
- Entlangführen einer ersten Flüssigkeit enthaltend eine Komponente A an einer Seite einer semipermeablen Membran und
- Entlangführen einer zweiten Flüssigkeit enthaltend eine Komponente B entlang einer anderen, vorzugsweise gegenüberliegenden Seite, der Membran,
wobei die Membran einen Durchtritt der Komponente A ausschließt, jedoch einen Durchtritt der Komponente B zulässt, und nach Durchtritt der Komponente B durch die Membran eine chemische Reaktion, vorzugsweise unter Ausbildung kovalenter Bindungen, zwischen der Komponente A und der Komponente B stattfindet.

Weiterhin betrifft die Erfindung ein Produkt, vorzugsweise in Form einer funktionalisierten hochmolekularen Komponente wie beispielsweise eines funktionalisierten Proteins, welches nach diesem Verfahren hergestellt oder herstellbar ist.

Des Weiteren betrifft die Erfindung die Verwendung eines Dialysators oder einer Dialysekassette sowie einer Dialysevorrichtung zur Durchführung des Verfahrens.

## Beschreibung

Die Erfindung betrifft ein Verfahren zur Herstellung eines Produktes, vorzugsweise in Form einer funktionalisierten hochmolekularen Komponente, mittels Dialyse, ein Produkt, welches nach einem solchen Verfahren hergestellt oder herstellbar ist sowie die Verwendung eines Dialyseapparates zur Durchführung eines solchen Verfahrens.

Die Herstellung von Arzneimitteln oder medizinisch wirksamen Zusammensetzungen wie beispielsweise der in der WO 2009/095223 A1 beschriebenen injizierbaren Zusammensetzungen muss aus Gründen der Qualitätssicherung der Herstellungsabläufe und -umgebung in der Regel in GMP-Reinräumen (Good Manufacturing Practice-Reinräumen) erfolgen.

Sehr häufig erfolgt die Herstellung von Arzneimitteln mittels offener Verfahrenstechniken, bei welchen einzelne Verfahrensschritte wie beispielsweise Herstellung, Aufreinigung und gegebenenfalls Reduktion von Probevolumina kompartimentiert bzw. räumlich getrennt voneinander ablaufen.

Derartige Verfahrenstechniken erschweren jedoch in erheblichem Maße eine Sterilitätskontrolle sowie eine Stabilisierung eines aseptischen Herstellungsprozesses. Die Durchführung steriler bzw. aseptischer Herstellungsprozesse wird durch die langen Herstellungszeiten, welche üblicherweise aus offenen Verfahrensführungen resultieren, zusätzlich erschwert.

Insgesamt erfordern die vorstehend beschriebenen Verfahren zur Herstellung von Produkten mit GMP-Qualität einen deutlich erhöhten Qualifizierungs- und Validierungsaufwand.

Aus der DE 693 30 179 T2 ist ein Verfahren zur Entfernung von unerwünschten oder potentiell schädlichen, proteingebundenen Stoffen (PBS) aus einer proteinhaltigen Flüssigkeit wie beispielsweise Plasma oder Blut mittels Dialyse bekannt.

### Aufgabe und Lösung

Es ist daher eine Aufgabe der Erfindung, ein Herstellungsverfahren für Produkte, bei welchen insbesondere die Erlangung einer GMP-Qualität erforderlich ist, bereitzustellen, wobei die einleitend, aus dem Stand der Technik bekannten Nachteile wenigstens weitgehend vermieden werden. Es ist es weiterhin eine Aufgabe der Erfindung, ein Produkt bereitzustellen, welches durch oder im Rahmen des erfindungsgemäßen Verfahrens hergestellt bzw. herstellbar ist. Außerdem ist es eine Aufgabe der Erfindung, eine Verwendung eines Dialysators oder einer Dialysekassette sowie einer Dialysevorrichtung bereitzustellen.

Diese Aufgabe wird erfindungsgemäß gelöst durch ein Verfahren mit den Merkmalen des unabhängigen Anspruchs 1, ein Produkt mit den Merkmalen des Anspruchs 14 sowie durch Verwendungen gemäß den Ansprüchen 15 und 16. Bevorzugte Ausführungsformen des Verfahrens sind Gegenstand der abhängigen Ansprüche 2 bis 13. Der Wortlaut sämtlicher Ansprüche wird hiermit durch ausdrückliche Bezugnahme zum Inhalt der vorliegenden Beschreibung gemacht.

Bei dem erfindungsgemäßen Verfahren handelt es sich um ein Verfahren zur Herstellung, insbesondere mit nachfolgender Aufreinigung und/oder Aufkonzentrierung, eines Produktes, vorzugsweise eines Produktes, welches GMP-Standards entspricht.

Bei dem Produkt handelt es sich bevorzugt um eine hochmolekulare und vorzugsweise funktionalisierte Komponente wie beispielsweise ein funktionalisiertes Protein.

Das Verfahren umfasst die folgenden Schritte:
- Entlangführen einer ersten Flüssigkeit enthaltend eine Komponente A an einer Seite einer semipermeablen Membran und
- Entlangführen einer zweiten Flüssigkeit enthaltend eine Komponente B entlang einer anderen, vorzugsweise gegenüberliegenden, Seite der Membran.

Die Membran schließt einen Durchtritt der Komponente A aus. Indes lässt die Membran einen Durchtritt der Komponente B zu.

Nach Durchtritt der Komponente B durch die Membran findet eine chemische Reaktion, vorzugsweise unter Ausbildung von kovalenten Bindungen, zwischen der Komponente A und der Komponente B statt.

Mit anderen Worten betrifft die vorliegende Erfindung somit ein auf einer Dialyse, d.h. einem Stofftransport über eine semipermeable Membran, beruhendes chemisches Herstellungsverfahren für Produkte.

Dabei kann bereits die zwischen den Komponenten A und B stattfindende Reaktion zu dem gewünschten Produkt führen.

Erfindungsgemäß kann es jedoch durchaus bevorzugt sein, dass für die Herstellung des Produktes die Reaktion mit einer weiteren Komponente erforderlich ist, worauf im Folgenden noch näher eingegangen wird.

Unter dem Ausdruck "semipermeable Membran" soll im Sinne der vorliegenden Erfindung eine Membran verstanden werden, welche "halbdurchlässig" oder "teilweise durchlässig" ist und - wie vorstehend ausgeführt - für die Komponente A undurchlässig, indes (wenigstens) für die Komponente B durchlässig ist.

Unter dem Ausdruck "funktionalisiert" soll im Sinne der vorliegenden Erfindung jeder abgeschlossene Vorgang verstanden werden, mittels dessen dem Produkt, beispielsweise durch Hinzufügen von Atomgruppierungen bzw. funktionellen Gruppen, eine Funktion verliehen wird, die das Produkt normalerweise nicht besitzt.

In einer bevorzugten Ausführungsform handelt es sich daher bei dem erfindungsgemäßen Verfahren um ein Verfahren zur Funktionalisierung, insbesondere Derivatisierung, eines Produktes, insbesondere einer hochmolekularen Komponente.

Unter dem Ausdruck "hochmolekulare Komponente" soll im Sinne der vorliegenden Erfindung zweckmäßigerweise eine Komponente verstanden werden, deren Molekulargewicht größer ist als die molekulare Ausschlussgrenze (molecular weight cut off, MWCO) der semipermeablen Membran.

Die Herstellung des Produktes umfasst - wie bereits erwähnt - bevorzugt dessen Aufreinigung und/oder Aufkonzentrierung.

Die zweite Flüssigkeit wird vorzugsweise im Gegenstrom zur ersten Flüssigkeit entlang der Membran geführt (bzw. umgekehrt). Die Anwendung des Gegenstromprinzips ermöglicht mit besonderem Vorteil eine Aufkonzentrierung des hergestellten Produktes sowie zudem eine Reduzierung der hierfür erforderlichen Flüssigkeitsmenge, beispielsweise Dialysepuffermenge. Ein weiterer Vorteil des Gegenstromprinzips liegt in der präzisen Einstellung des osmotischen Druckes. Außerdem lässt sich die Effektivität des Stoffübergangs über der Membran mittels des Gegenstromprinzips steigern.

Die erste Flüssigkeit und die zweite Flüssigkeit werden in zweckmäßigen Ausführungsformen jeweils mittels Pumpen, vorzugsweise Schlauchpumpen, entlang der Membran geführt. Die Verwendung von Schlauchpumpen ermöglicht in besonders vorteilhafter Weise die Erzeugung von gegenläufigen Strömen der ersten Flüssigkeit und der zweiten Flüssigkeit.

Um die Reaktion zwischen den Komponenten A und B zu kontrollieren, kann es erfindungsgemäß bevorzugt sein, die Konzentration der Komponente A in der ersten Flüssigkeit und/oder die Konzentration der Komponente B in der zweiten Flüssigkeit zu variieren, insbesondere graduell zu variieren (Gradientenmodus).

Beispielsweise kann zur Beschleunigung der zwischen den Komponenten A und B stattfindenden Reaktion die Konzentration der Komponente A in der ersten Flüssigkeit und/oder die Konzentration der Komponente B in der zweiten Flüssigkeit erhöht, insbesondere graduell erhöht (Gradientenmodus), werden.

Die erste Flüssigkeit und die zweite Flüssigkeit werden jeweils bevorzugt über einen geschlossenen Kreislauf an der Membran entlang geführt. So kann die erste Flüssigkeit über einen ersten geschlossenen Kreislauf und die zweite Flüssigkeit über einen zweiten geschlossenen Kreislauf an der Membran entlang geführt werden. Dadurch können insbesondere die im Zusammenhang mit offenen Verfahren einleitend diskutierten Nachteile in Bezug auf Sterilitätskontrolle und Stabilisierung eines aseptischen Herstellungsprozesses vermieden werden.

Zur Erzeugung eines Druckgradienten über der Membran kann es in einer weiteren Ausführungsform vorgesehen sein, dass die erste Flüssigkeit, insbesondere in dem im vorherigen Absatz erwähnten ersten Kreislauf, in einem Saugmodus zirkuliert und die zweite Flüssigkeit, insbesondere in dem im vorherigen Absatz erwähnten zweiten Kreislauf, in einem Druckmodus geführt wird.

In besonders vorteilhaften Ausführungsformen erfolgt die Herstellung, insbesondere Funktionalisierung, sowie eine sich anschließende Aufreinigung und/oder Aufkonzentrierung des Produktes über die im vorherigen Absatz beschriebene geschlossene Kreislaufführung.

Die erfindungsgemäß vorgesehene Membran ist üblicherweise Bestandteil eines Membranreaktors. Als Membranreaktor wird vorzugsweise ein Dialysator verwendet. Mit anderen Worten wird die Herstellung des Produktes vorzugsweise in einem Dialysator durchgeführt, welcher die semipermeable Membran enthält.

Bei dem Dialysator kann es sich insbesondere um einen Kapillardialysator handeln. Ein Kapillardialysator besteht üblicherweise aus einem Gehäuse, in dem eine große Anzahl, beispielsweise bis zu 10 000 Hohlfasern, parallel zueinander beziehungsweise im Wesentlichen parallel zueinander angeordnet sind. Die Hohlfasern können eine Länge von 15 cm bis 30 cm, insbesondere 20 cm bis 25 cm, besitzen. Weiterhin können die Hohlfasern einen Durchmesser von 200 µm bis 400 µm, insbesondere von ca. 300 µm, besitzen. Die Hohlfasern können außerdem eine Wandstärke von 30 µm bis 50 µm, insbesondere von ca. 40 µm, besitzen.

Bei Verwendung eines Kapillardialysators erfolgt die Herstellung des Produktes vorzugsweise in der Weise, dass die erste, die Komponente A enthaltende Flüssigkeit innerhalb der Hohlfasern und die zweite, die Komponente B enthaltende Flüssigkeit außerhalb der Hohlfasern, vorzugsweise im Gegenstrom, geführt werden. Durch die Verwendung eines Kapillardialysators ist eine aufgrund der durch die große Anzahl von Hohlfasern bedingten großen spezifischen Membranoberfläche rasche und insbesondere effiziente Herstellung des Produktes möglich.

Der in den vorherigen Ausführungsformen beschriebene Dialysator kann insbesondere in Form einer Dialysekassette vorliegen.

Besonders bevorzugt wird die Herstellung des Produktes mittels eines Dialyseapparates bzw. -gerätes durchgeführt. Die Verwendung eines Dialysegerätes, wie es üblicherweise zur Blutwäsche eingesetzt wird, hat den großen Vorteil, dass es sich hierbei um ein bereits auf medizintechnischem Gebiet qualifiziertes bzw. validiertes Gerät handelt, auf dessen Qualifizierungs- bzw. Validierungsunterlagen daher ohne Weiteres zurückgegriffen werden kann. Obendrein ermöglicht die Verwendung eines Dialysegerätes eine Teilautomatisierung des Herstellungsprozesses.

Hinsichtlich geeigneter Dialysegeräte bestehen unter erfindungsgemäßen Gesichtspunkten prinzipiell keine Limitierungen.

Üblicherweise besitzt ein erfindungsgemäß geeignetes Dialysegerät jedoch als zentrale Einrichtung einen Dialysator, in dem über eine semipermeable Membran ein Stoffaustausch in der vorstehend beschriebenen Art und Weise (Durchtritt von Komponente B, nicht jedoch von Komponente A) zwischen der ersten und zweiten Flüssigkeit stattfinden kann. Vorzugsweise wird die erste, die Komponente A enthaltende Flüssigkeit über ein als extrakorporalen Blutkreislauf bezeichnetes Kreislaufsystem und die zweite, die Komponente B enthaltende Flüssigkeit über ein als Dialysatkreislauf bezeichnetes Kreislaufsystems entlang der Membran geführt. Mit dem Aufbau von Dialysegeräten ist der Fachmann hinreichend vertraut, so dass von weitergehenden Ausführungen Abstand genommen wird.

Insgesamt erfüllt ein Dialysegerät daher alle Systemvoraussetzungen zur Durchführung des erfindungsgemäßen Verfahrens.

Im Gegensatz zu einer manuellen Dialyse hat die Verwendung eines Dialysegerätes den weiteren Vorteil, dass der Flüssigkeitsaufwand, insbesondere hinsichtlich der ersten Flüssigkeit, reduziert werden kann.

Die bei dem erfindungsgemäßen Verfahren zum Einsatz kommende Membran besitzt vorzugsweise eine Ausschlussgrenze (molecular weight cut off, MWCO) von 2 kDa (kiloDalton) bis 20 kDa (kiloDalton), insbesondere 3 kDa (kiloDalton) bis 18 kDa (kiloDalton), bevorzugt 5 kDa (kiloDalton) bis 15 kDa (kiloDalton).

Weiterhin kann die Membran aus Materialen hergestellt sein, welche ausgewählt sind aus der Gruppe umfassend Keramiken, Graphit, Metalle, Metalloxide, Polymere, insbesondere organische Polymere, und Mischungen davon.

Beispielsweise kann die Membran aus einem Polymer hergestellt sein, welches ausgewählt ist aus der Gruppe umfassend Polysulfone, Polyamide, Polycarbonate, Polyester, Acrylnitrilpolymere, Polyvinylalkohole, Acrylatpolymere, Methacrylatpolymere, Celluloseacetatpolymere und Mischungen davon. Erfindungsgemäß verwendbare Membranen sind dem Fachmann als solche bekannt und beispielsweise in Kirk-Othmer, Encyclopedia of Chemical Technology, 3. Auflage, Band 7 (1979), 564-579, insbesondere 574-575, Band 12 (1980), 492-517 und Band 15 (1981), 92-131 beschrieben. Darüber hinaus sind geeignete Membranen auch in Ullmann's Encyclopedia of Industrial Chemistry, 5. Auflage, Band A 16 (1990), 187-263 beschrieben.

Die zweite Flüssigkeit kann in einer weiteren Ausführungsform neben der Komponente B eine weitere Komponente C enthalten. Alternativ kann nach der zweiten, die Komponente B enthaltenden Flüssigkeit eine dritte Flüssigkeit an der Membran entlang geführt werden, welche eine weitere Komponente C enthält. Bevorzugt ist die Membran bei den in diesem Absatz beschriebenen Ausführungsformen auch für die Komponente C durchlässig.

In einer weitergehenden Ausführungsform findet nach Durchtritt der gegebenenfalls zusätzlich vorgesehenen Komponente C durch die Mem-bran zwischen der Komponente A und der Komponente C ebenfalls eine chemische Reaktion, vorzugsweise unter Ausbildung von kovalenten Bindungen, statt.

Bevorzugt reagieren die Komponente B und die gegebenenfalls zusätzlich vorgesehene Komponente C an unterschiedlichen Stellen der Komponente A. Insbesondere reagieren die Komponenten B und C mit verschiedenen Atomgruppen, bevorzugt funktionellen Gruppen, der Komponente A.

Bei der Komponente B und/oder der gegebenenfalls zusätzlich vorgesehenen Komponente C handelt es sich bevorzugt um eine Komponente mit einem Molekulargewicht < 5 kDa, insbesondere < 3 kDa, bevorzugt < 2 kDa.

Bei der Komponente B und/oder der gegebenenfalls zusätzlich vorgesehenen Komponente C handelt es sich vorzugsweise um eine thiolreaktive Verbindung, d.h. um eine Verbindung, welche befähigt ist, mit Thiol- und/oder Thiolatgruppen der Komponente A zu reagieren. Die Reaktion verläuft vorzugsweise selektiv und kann insbesondere auf einer Michael-Addition beruhen.

Geeignete thiolreaktive Verbindungen können aus der Gruppe umfassend Maleimidverbindungen, Vinylsulfonverbindungen, Acrylatverbindungen, Alkylhalogenidverbindungen, Azirinverbindungen, Pyridinverbindungen, Thionitrobenzoesäureverbindungen, Arylverbindungen, Derivate davon und Mischungen davon ausgewählt sein.

Vorzugsweise handelt es sich bei der Komponente B und/oder der gegebenenfalls zusätzlich vorgesehenen Komponente C um eine Maleimidverbindung bzw. ein Maleimidderivat.

Besonders bevorzugt handelt es sich bei der Komponente B um 3-Maleimidopropionsäure (bzw. N-Maleoyl-β-Alanin).

Alternativ oder in Kombination zu den vorangegangenen Ausführungsformen kann es sich bei der Komponente B und/oder der gegebenenfalls zusätzlich vorgesehenen Komponente C um eine aminogruppenreaktive Verbindung handeln. Unter einer aminogruppenreaktiven Verbindung soll im Sinne der vorliegenden Erfindung eine Verbindung verstanden werden, welche befähigt ist, mit Aminogruppen, vorzugsweise primären Aminogruppen, der Komponente A zu reagieren.

Bevorzugt handelt es sich bei der Komponente B und/oder der gegebenenfalls zusätzlich vorgesehenen Komponente C um eine aminogruppen- und thiolreaktive Verbindung.

Insbesondere kann es sich bei der Komponente B und/oder der gegebenenfalls zusätzlich vorgesehenen Komponente C um einen Aktivester handeln. Unter einem Aktivester soll im Sinne der vorliegenden Erfindung ein Ester mit einer aktivierten Acylgruppe, d.h. ein Ester mit erhöhtem Acylierungspotential, verstanden werden. Das Aktivierungsvermögen beruht vorzugsweise auf der Anwesenheit einer guten Abgangsgruppe, die mit dem Acylkohlenstoffatom kovalent verbunden ist.

Erfindungsgemäß ist es insbesondere bevorzugt, wenn es sich bei der Komponente B und/oder der gegebenenfalls zusätzlich vorgesehenen Komponente C um einen maleimidfunktionalisierten Aktivester, d.h. um einen Aktivester mit einer Maleimideinheit, handelt.

Vorzugsweise handelt es sich bei der Komponente B und/oder der gegebenenfalls zusätzlich vorgesehenen Komponente C um 3-Maleimido-propionsäure-N-Hydroxysuccinimidester.

Die erfindungsgemäß vorgesehenen Flüssigkeiten können ausgewählt sein aus der Gruppe umfassend Lösungen, Dispersionen und Suspensionen. Als Lösungs-, Dispersions- oder Suspensionsmittel kommen insbesondere Wasser und/oder Dimethylformamid in Betracht.

Erfindungsgemäß besonders bevorzugt ist die Verwendung einer Lösung enthaltend die Komponente A als erste Flüssigkeit, einer Lösung enthaltend die Komponente B als zweite Flüssigkeit sowie gegebenenfalls einer Lösung enthaltend die Komponente C als eine gegebenenfalls zusätzlich vorgesehene dritte Flüssigkeit.

Die Komponente A kann in der ersten Flüssigkeit in einer Konzentration von 100 µmol/l bis 1.5 mmol/l, insbesondere 200 µmol/l bis 1 mmol/l, bevorzugt 300 µmol/l bis 800 µmol/l, enthalten sein.

Die Komponente B kann in der zweiten Flüssigkeit in einer Konzentra-tion von 35 mmol/l bis 530 mmol/l, insbesondere 70 mmol/l bis 350 mmol/l, bevorzugt 105 mmol/l bis 280 mmol/l, enthalten sein.

Die gegebenenfalls vorgesehene weitere Komponente C kann in der zweiten Flüssigkeit oder in einer dritten Flüssigkeit ebenfalls in einer Konzentration von 35 mmol/l bis 530 mmol/l, insbesondere 70 mmol/l bis 350 mmol/l, bevorzugt 105 mmol/l bis 280 mmol/l, enthalten sein.

Die Komponenten A, B und/oder die gegebenenfalls vorgesehene Komponente C können in einer Strömungsrate von 25 ml/min bis 500 ml/min, insbesondere 100 ml/min bis 400 ml/min, bevorzugt 200 ml/min bis 300 ml/min, pro Quadratmeter Membranfläche an der Membran entlang geführt werden.

Das Verfahren kann in vorteilhaften Ausführungsformen bei Raum- bzw. Umgebungstemperatur, insbesondere in einem Temperaturbereich von 20 °C bis 30 °C, durchgeführt werden.

Weiterhin kann das Verfahren in einem Zeitraum von 15 min bis 5 h, insbesondere 30 min bis 3 h, bevorzugt 45 min bis 2 h, durchgeführt werden.

Die Komponente A ist vorzugsweise eine hochmolekulare Komponente. Beispielsweise kann die Komponente A ein Molekulargewicht von 10 kDa bis 90 kDa, insbesondere 15 kDa bis 80 kDa, bevorzugt 20 kDa bis 70 kDa, aufweisen.

Die Komponente A kann weiterhin ein synthetisches oder biologisches, insbesondere natürlich vorkommendes oder rekombinant hergestelltes, Polymer sein.

Die Komponente A kann in einer weiteren Ausführungsform humanen oder xenogenen, insbesondere bovinen, porcinen oder equinen, Ursprungs sein.

Beispielsweise kann die Komponente A ausgewählt sein aus der Gruppe umfassend Proteine, insbesondere Serumproteine wie beispielsweise Albumin, Enzyme, Antikörper, extrazelluläre Proteine wie beispielsweise Collagen, Elastin, Retikulin, Fibronektin oder dergleichen, Hormone, Wachstumsfaktoren, Cytokine, Polysaccharide, insbesondere Mucopolysaccharide wie beispielsweise Hyaluronsäure, Salze davon, Derivate davon, Konjugate davon und Mischungen davon.

In einer besonders bevorzugten Ausführungsform handelt es sich bei der Komponente A um Albumin. Das Albumin besitzt bevorzugt ein Molekulargewicht von 66 kDa bis 67 kDa, insbesondere von 66 kDa.

Bevorzugt handelt es sich bei der Komponente A um Serumalbumin, insbesondere bovines oder humanes Serumalbumin.

Das erfindungsgemäß herzustellende Produkt kann grundsätzlich aus der Gruppe umfassend Arzneimittel, pharmazeutische Verbindungen, Medizinprodukte, Lebensmittel und Kosmetikprodukte ausgewählt sein.

Vorzugsweise ist das erfindungsgemäß herzustellende Produkt ausgewählt aus der Gruppe umfassend funktionalisierte Proteine wie beispielsweise funktionalisiertes Albumin, funktionalisierte Enzyme, funktionalisierte Antikörper, funktionalisierte extrazelluläre Proteine, funktionalisierte Hormone, funktionalisierte Wachstumsfaktoren, funktionalisierte Cytokine, funktionalisierte Polysaccharide, funktionalisierte Mucopolysaccharide, Salze davon und Mischungen davon.

Bevorzugt handelt es sich bei dem herzustellenden Produkt um ein Maleimidfunktionalisiertes Protein, insbesondere um Maleimid-funktionalisiertes Albumin.

Besonders bevorzugt handelt es sich bei dem herzustellenden Produkt um das in der einleitend erwähnten WO 2009/095223 A1 beschriebene hydrophile Polymer, insbesondere funktionalisierte bzw. modifizierte Albumin, weswegen der Offenbarungsgehalt der PCT-Offenlegungsschrift insoweit durch ausdrückliche Bezugnahme zum Inhalt der vorliegenden Beschreibung gemacht wird.

Die erste, zweite und/oder gegebenenfalls vorgesehene dritte Flüssigkeit liegt in zweckdienlichen Ausführungsformen jeweils als wässrige Pufferlösung, insbesondere wässrige Phosphatpufferlösung, und/oder wässrige Elektrolytlösung vor.

Insbesondere können in der ersten, zweiten und/oder gegebenenfalls vorgesehenen dritten Flüssigkeit Salze vorgesehen sein, die ausgewählt sind aus der Gruppe umfassend Natriumchlorid, Kaliumchlorid, Natriumdihydrogenphosphat, Dinatriumhydrogenphosphat, Natriumphosphat, Kaliumdihydrogenphosphat, Dikaliumhydrogenphosphat" Ka-lilumphosphat, Magnesiumchlorid, Calciumchlorid, Natriumlactat, Glucosemonohydrat und Mischungen davon.

Ein zweiter Aspekt der Erfindung betrifft ein Produkt, vorzugsweise in Form einer funktionalisierten hochmolekularen Komponente wie beispielsweise eines funktionalisierten Proteins, welches hergestellt oder herstellbar ist nach einem der in den vorhergehenden Ausführungsformen beschriebenen Verfahren. Bezüglich weiterer Merkmale und Vorteile des Produktes, insbesondere des Verfahrens zu seiner Herstellung, wird zur Vermeidung unnötiger Wiederholungen vollständig auf die vorliegende Beschreibung Bezug genommen.

In einem dritten Aspekt betrifft die Erfindung die Verwendung eines Dialysators oder einer Dialysekassette zur Durchführung des erfindungsgemäßen Verfahrens. Bezüglich weiterer Merkmale und Vorteile des Dialysators oder der Dialysekassette sowie des Verfahrens wird ebenfalls vollständig auf die vorliegende Beschreibung Bezug genommen.

Schließlich betrifft die Erfindung in einem vierten Aspekt die Verwendung einer Dialysevorrichtung oder eines Dialyseapparates bzw. -gerätes zur Durchführung des erfindungsgemäßen Verfahrens. Bezüglich weiterer Merkmale und Vorteile der Dialysevorrichtung oder des Dialyseapparates bzw. -gerätes sowie des Verfahrens wird ebenso vollständig auf die vorliegende Beschreibung Bezug genommen.

An dieser Stelle sollen die Vorteile der Erfindung noch einmal wie folgt zusammengefasst werden:
➢ Das erfindungsgemäße Verfahren erlaubt mit besonderem Vorteil eine vollständig geschlossene Verfahrensführung, wodurch eine sichere aseptische Produktherstellung möglicht ist.
➢ Das erfindungsgemäße Verfahren erlaubt obendrein eine Aufreinigung und/oder Aufkonzentrierung des hergestellten Produktes.
➢ Insbesondere ist es möglich, die Herstellung des Produktes einschließlich einer sich daran anschließenden Aufreinigung und/oder Aufkonzentrierung desselben in einem geschlossenen System durchzuführen.
➢ Besonders vorteilhaft ist, dass das erfindungsgemäße Verfahren mittels eines Dialyseapparates bzw. -gerätes durchgeführt werden kann. Dialyseapparate bzw. -geräte sind auf dem medizinischen Gebiet hinreichend erprobt, so dass umfangreiche Qualifizierungs- bzw. Validierungsunterlagen zur Verfügung stehen, welche eine erfolgreiche Verfahrensführung garantieren.
   Außerdem kann die Verwendung von mobilen Dialyseapparaten zu einer weiteren Vereinfachung und insbesondere Flexibilisierung des Verfahrens beitragen.
➢ Ein weiterer Vorteil besteht darin, dass das erfindungsgemäße Verfahren einen in Bezug auf das herzustellende Produkt schonenden Prozess darstellt, der insbesondere bei Raumtemperatur ablaufen kann.
➢ Weiterhin vorteilhaft sind die (deutlich) kürzeren Herstellungszeiten, wodurch sich der aseptische Sicherheitsstandard zusätzlich erhöht.
➢ Vorteilhaft ist des Weiteren, dass das erfindungsgemäße Verfahren das Arbeiten mit mittelgroßen Ansätzen erlaubt. Bislang können Ansätze nämlich nur im Labormaßstab oder in großtechnischen Anlagen gefahren werden.
➢ Ferner ist als Vorteil hervorzuheben, dass das erfindungsgemäße Verfahren mit deutlich geringeren Volumina an Dialysepufferlösungen auskommt als gattungsgemäße Verfahren.
Schließlich ist von Vorteil, dass das erfindungsgemäße Verfahren eine automatisierbare und insbesondere skalierbare Prozessführung erlaubt.

Weitere Merkmale und Vorteile der Erfindung ergeben sich aus der nachfolgenden Beschreibung einer bevorzugten Ausführungsform anhand eines Ausführungsbeispiels. Es versteht sich, dass die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

### Figurenbeschreibung

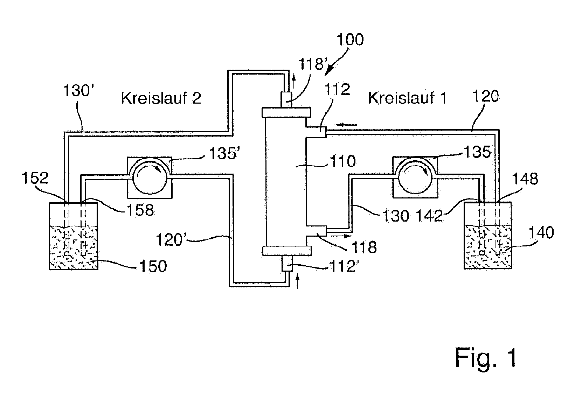

Fig. 1 zeigt schematisch den Aufbau einer Dialysevorrichtung 100 zur Durchfü h-rung des erfindungsgemäßen Verfahrens.

Die Vorrichtung 100 umfasst einen Membranreaktor 110 sowie zwei Vorratsbehältnisse 140 und 150.

Der Membranreaktor 110 enthält eine semipermeable Membran. Die Membran ist zum Beispiel für eine Komponente A undurchlässig, während sie für eine Komponente B durchlässig ist. Bei dem Membranreaktor 110 handelt es sich in der Regel um einen Dialysator bzw. eine Dialysekassette.

Das Behältnis 140 ist für eine Bevorratung einer ersten Flüssigkeit enthaltend eine Komponente A vorgesehen. Die Komponente A kann ein Protein, insbesondere Albumin, sein.

Das Behältnis 150 ist für eine Bevorratung einer zweiten Flüssigkeit enthaltend eine Komponente B vorgesehen. Die Komponente B kann eine aminogruppen- und thiolreaktive Verbindung, wie beispielsweise 3-Maleimido-propionsäure-N-Hydroxysuccinimidester, sein.

Das Vorratsbehältnis 140 weist einen Einlass 142 und einen Auslass 148 auf.

Entsprechend weist das Vorratsbehältnis 150 einen Einlass 152 und einen Auslass 158 auf.

Der Auslass 148 ist über eine Zuführleitung 120 mit einem Einlass 112 des Membranreaktors 110 verbunden, während der Einlass 142 über eine Abführleitung 130 mit einem Auslass 118 des Membranreaktors 110 verbunden ist.

Entsprechend ist der Auslass 158 über eine Zuführleitung 120' mit einem Einlass 112' des Membranreaktors 110 und der Einlass 152 über eine Abführleitung 130' mit einem Auslass 118' des Membranreaktors 110 verbunden.

Zwischen dem Auslass 118 und dem Einlass 142 einerseits und dem Auslass 158 und dem Einlass 112' andererseits ist vorzugsweise eine Pumpe 135 bzw. 135' angeordnet. Bei der Pumpe kann es sich beispielsweise um eine peristaltische Pumpe handeln.

Der Membranreaktor 110 bildet zusammen mit Leitungen 120 und 130 sowie dem Behältnis 140 einen ersten geschlossenen Kreislauf und zusammen mit den Leitungen 120' und 130' sowie dem Behältnis 150 einen zweiten geschlossenen Kreislauf.

Es folgt eine beispielhafte Erläuterung des erfindungsgemäßen Verfahrens anhand der in Figur 1 dargestellten Vorrichtung:

Über die Zuführleitung 120 wird die in dem Behältnis 140 bevorratete, die Komponente A enthaltende erste Flüssigkeit dem Membranreaktor 110 zugeführt und innerhalb des Membranreaktors 110 entlang von dessen semipermeabler Membran entlanggeführt. Über die Abführleitung 130 wird die erste Flüssigkeit wieder aus dem Membranreaktor 110 abgeführt und in das Vorratsbehältnis 140 zurückgeführt. Mittels der Pumpe 135 kann die erste Flüssigkeit kontinuierlich innerhalb des ersten Kreislaufs geführt werden.

Entsprechend wird über die Zuführleitung 120' die in dem Behältnis 150 bevorratete, die Komponente B enthaltende zweite Flüssigkeit dem Membranreaktor 110 zugeführt und innerhalb des Membranreaktors 110 entlang von dessen semipermeabler Membran entlanggeführt. Über die Abführleitung 130' wird die zweite Flüssigkeit wieder aus dem Membranreaktor 110 abgeführt und in das Vorratsbehältnis 150 zurückgeführt. Mittels der Pumpe 135' kann die zweite Flüssigkeit kontinuierlich innerhalb des zweiten Kreislaufs geführt werden.

Während des Entlangführens der zweiten Flüssigkeit entlang der im Membranreaktor 110 enthaltenen semipermeablen Membran passiert ein Teil der entlanggeführten Stoffmenge von Komponente B die Membran und reagiert auf der gegenüberliegenden Membranseite mit der dort entlanggeführten Komponente A. Die Reaktion zwischen den Komponenten A und B beruht dabei vorzugsweise auf der Ausbildung kovalenter Bindungen.

Handelt es sich, wie oben erwähnt, im Falle der Komponente A um ein Protein, beispielsweise Albumin, und im Falle der Komponente B um 3-Maleimidopropionsäure-N-Hydroxysuccinimidester, so reagieren Maleimido-propionsäure-N-Hydroxysuccinimidestermoleküle nach ihrem Durchtritt durch die semipermeable Membran bevorzugt mit primären Aminogruppen des Proteins, wodurch maleimidfunktionalisiertes Protein entsteht.

### Ausführungsbeispiel

### 1. Material und Methoden

### 1.1 Lösungen

Albuminlösung: 10 mg/ml humanes Serumalbumin (Fraction V, Cal-biochem, Kat.-Nr. 12668) in 200 ml 0.2 mol/l Na₂HPO₄, 0.4 mol/l Borsäure, mit NaOH auf pH 8.1 eingestellt.

SMP-Lösung 1: 2000 mg 3-Maleimidopropionsäure-N-Hydroxysuccinimid-Ester (Obiter Research, Champaign IL, USA, Kat.-Nr. OBT-104) in 25 ml N,N-Dimethylformamid (Sigma-Aldrich, Kat.-Nr. 22, 705-6) gelöst.

SMP-Lösung 2: 250 ml 50 mmol/l Na-Citrat (pH 3.6), 10% (v/v) SMP-Lösung 1.

Natriumacetat: 0.3 M NaOOCCH₃ (pH 4.7)
Natriumdihydrogenphosphat: 10 mmol/l NaH₂PO₄

### 1.2 Geräte

Dialyse-Kassette: H.E.L.P. Ultrafilter SMC 1.8
Schlauchmaterial: Pharmed^{®} NSF-51
Dialyse-Kassette, Schlauchmaterial und Schlauchverbinder wurden von P. Mandry (MAT Adsorption Technologies GmbH & Co. KG, Obernburg) bereitgestellt.

Gelchromatographische Untersuchungen wurden mit einem Smart Chromatographie-System (Pharmacia) und einer Superose 6 10/300 GL Säule (GE Healthcare) durchgeführt. UV-Spektroskopie erfolgte mit einem Spectronic Genesys 2-Gerät.

### 1.3 Gelbildung

10xCB (pH 7.2) und PEG-Link (beide Cellendes GmbH, Reutlingen) wurden als Puffer bzw. als Vernetzer zur Testung der Gelbildung des maleimidfunktionalisierten Albumins eingesetzt.

### 2. Durchführung

Die Funktionalisierung wurde in einem Membranreaktor bei 4°C durchgeführt, welcher Bestandteil eines Systems ist, wie in Figur 1 dargestellt.

Zur Funktionalisierung von Albumin mit Maleimidgruppen wurden 200 ml Albuminlösung im ersten Kreislauf (siehe Fig. 1) mit einer Flussrate von 35 ml/min zirkuliert. Im zweiten Kreislauf (siehe Fig. 1) wurden dagegen 52 ml SMP-Lösung 2 mit einer Flussrate von 100ml/min für 30 min zirkuliert.

Da im ersten Kreislauf die Albuminlösung im Saugmodus durch die Dialysekassette und im zweiten Kreislauf die SMP-Lösung 2 im Druckmodus zirkuliert wurde, entstand ein Druckgradient über der Dialysemem-bran. Aufgrund dieses Druckgefälles war nach 30 min der Vorratsbehälter für die SMP-Lösung 2 vollständig entleert.

Im Anschluss an die Funktionalisierung wurde der leere Vorratsbehälter für die SMP-Lösung 2 durch einen Behälter mit einem Liter Natrium-acetatlösung ersetzt. Diese Lösung wurde nicht zirkuliert, sondern mit einer Flussrate von 100 ml/min durch den Membranreaktor gepumpt und in einem separaten Gefäß gesammelt.

Da das Volumen der Albuminlösung nach dem Ansäuern auf ca. 600 ml angestiegen war, wurde die Konfiguration des Membranreaktors geändert, um eine Konzentrierung der Albuminlösung zu ermöglichen. Die Pumprichtung beider peristaltischer Pumpen wurde umgekehrt, wodurch auch der Druckgradient über die Membran umgekehrt wurde. Dieser Druckgradient wurde durch eine einstellbare Schlauchklemme, die im ersten Kreislauf einsetzt wurde, um einen Rückdruck in den Membranreaktor zu erzeugen, noch verstärkt. Die durch den Druckgradienten von Kreislauf 1 in Kreislauf 2 übertretende Flüssigkeit wurde bei einer Pumpgeschwindigkeit von 20 ml/min gesammelt. In diesem Modus wurde der Reaktor für 40 min betrieben, bis dass Volumen der Albuminlösung im Vorratsgefäß von Kreislauf 2 auf 200 ml reduziert war.

Die Schlauchklemme im Kreislauf 1 wurde wieder entfernt und zur Reinigung der Albuminlösung wurde die Natriumdihydrogenphosphat-Lösung mit einer Flussrate von 20 ml/min durch den Kreislauf 2 gepumpt. Das Eluat aus Kreislauf 2 wurde separat gesammelt. Nach Durchfluss von 1,8 Liter Reinigungslösung wurde aus dem Albuminkreislauf eine Probe für die Analyse entnommen, und die Reinigung wurde fortgesetzt, bis insgesamt 3 Liter Natriumdihydrogenphosphat durch den Reaktor gepumpt waren.

Die Konzentrierung der Albuminlösung nach der Reinigung erfolgte in derselben Konfiguration wie bei der ersten Konzentrierung. Der Reaktor wurde in diesem Modus betrieben, bis das Vorratsgefäß der Albuminlösung komplett entleert war. Im Anschluss wurde der Reaktor entleert, wobei 90 ml Albuminlösung erhalten wurden.

Zur Bestimmung der Reinheit wurden das ursprüngliche Albumin und das Material nach den verschiedenen Prozessschritten jeweils mittels Gelfiltrationschromatographie untersucht, um Kontaminationen mit niedermolekularen Substanzen nachzuweisen. Das ursprünglich eingesetzte Albumin wies nur geringe Kontaminationen mit niedermolekularen Substanzen auf. Nach der Prozessierung mit der SMP-Lösung 2 konnte dagegen ein starkes Absorptionssignal im niedermolekularen Bereich gemessen werden, welches von Maleimid-NHS-Gruppen und dem Citrat aus der SMP-Lösung 2 erzeugt wurde. Nach Reinigung mit 1,8 Liter Na-triumdihydrogenphosphat war dieses Signal bereits wesentlich schwächer und im Endprodukt war das Signal von niedermolekularen Substanzen unter 1% des Signals von Maleimid-Albumin gesunken.

Die Messungen der Lichtabsorption der ursprünglichen und der prozessierten Albuminlösung zeigten, dass durch den Membranreaktorprozess jedes Albumin-Molekül mit 16,3 Maleimidgruppen funktionalisiert wurde. Die Ausbeute betrug 57% des ursprünglich eingesetzten Albumins (siehe unten stehende Tabelle 1).

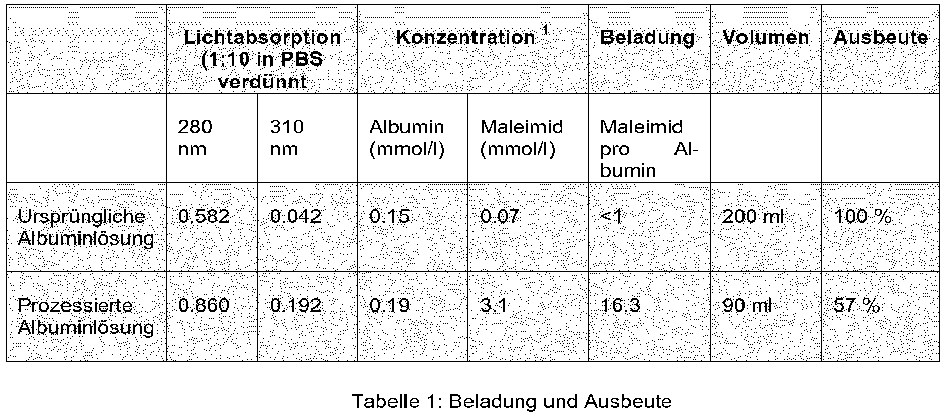

Zum Nachweis der Gelbildung wurde Maleimid-Albumin nach der Prozessierung im Membranreaktor zunächst mittels Gefriertrocknung 5-fach konzentriert. Zur Testung der Gelbildung wurden 3 µl 10-fach Puffer (pH 7.2), 7µl Wasser, 10 µl der konzentrierten Albuminlösung und 10 µl Bis-thio-Polyethylenglykol (20 mmol/L Thiolgruppen) miteinander vermischt. Innerhalb von 20 s nach Vermischung bildete sich aus dieser Lösung ein Hydrogel.

### 3. Zusammenfassung

Es wurde die kovalente Ankopplung von Maleimidgruppen an Albumin in einem Membranreaktor evaluiert. Als Membranreaktor wurde dabei eine Dialysekassette, wie sie bei der Blutreinigung eingesetzt wird, verwendet. Alle bei der Ankopplung notwendigen Schritte zur Funktionalisierung, Reinigung und Konzentrierung konnten mit dem Reaktorsystem durchgeführt werden, wobei die Albuminlösung während des gesamten Prozesses in einem stets geschlossenen Kreislauf verblieb. Es konnten bei diesem Prozess 16,3 Maleimidgruppen pro Albuminmolekül gekoppelt werden. Die Albuminausbeute betrug 57% und die Reinheit war über 99%. Das in dieser Weise hergestellte Maleimid-Albumin ist zur Gelbildung mit geeigneten thiolhaltigen Vernetzern geeignet.

## Patentansprüche

1. Verfahren zur Herstellung eines Produktes, vorzugsweise in Form einer funktionalisierten hochmolekularen Komponente wie beispielsweise eines funktionalisierten Proteins, umfassend die folgenden Schritte:
- Entlangführen einer ersten Flüssigkeit enthaltend eine Komponente A an einer Seite einer semipermeablen Membran und
- Entlangführen einer zweiten Flüssigkeit enthaltend eine Komponente B entlang einer anderen, vorzugsweise gegenüberliegenden, Seite der Membran,
wobei die Membran einen Durchtritt der Komponente A ausschließt, jedoch einen Durchtritt der Komponente B zulässt, und nach Durchtritt der Komponente B durch die Membran eine chemische Reaktion, vorzugsweise unter Ausbildung kovalenter Bindungen, zwischen der Komponente A und der Komponente B stattfindet.

2. Verfahren nach Anspruch 1, **dadurch gekennzeichnet, dass** die Herstellung des Produktes dessen Aufreinigung und/oder Aufkonzentrierung umfasst.

3. Verfahren nach Anspruch 1 oder 2, **dadurch gekennzeichnet, dass** die zweite Flüssigkeit im Gegenstrom zur ersten Flüssigkeit entlang der Membran geführt wird.

4. Verfahren nach einem der vorhergehenden Ansprüche, **dadurch gekennzeichnet, dass** die erste und zweite Flüssigkeit jeweils über einen geschlossenen Kreislauf an der Membran entlang geführt werden.

5. Verfahren nach einem der vorhergehenden Ansprüche, **dadurch gekennzeichnet, dass** die Herstellung des Produktes in einem die Membran enthaltenden Dialysator durchgeführt wird.

6. Verfahren nach einem der vorhergehenden Ansprüche, **dadurch gekennzeichnet, dass** die Herstellung des Produktes mittels einer Dialysevorrichtung oder eines Dialyseapparates bzw. -gerätes durchgeführt wird.

7. Verfahren nach einem der vorhergehenden Ansprüche, **dadurch gekennzeichnet, dass** die zweite Flüssigkeit neben der Komponente B eine membrangängige Komponente C enthält oder nach der zweiten Flüssigkeit eine dritte Flüssigkeit enthaltend eine membrangängige Komponente C an der Membran entlang geführt wird.

8. Verfahren nach Anspruch 7, **dadurch gekennzeichnet, dass** nach Durchtritt der Komponente C durch die Membran zwischen der Komponente A und der Komponente C ebenfalls eine chemische Reaktion, vorzugsweise unter Ausbildung kovalenter Bindungen, stattfindet, wobei die Komponente C bevorzugt mit anderen funktionellen Gruppen der Komponente A reagiert als die Komponente B.

9. Verfahren nach einem der vorhergehenden Ansprüche, **dadurch gekennzeichnet, dass** die Komponente A ausgewählt ist aus der Gruppe umfassend Proteine, Enzyme, Antikörper, extrazelluläre Proteine, Hormone, Wachstumsfaktoren, Cytokine, Polysaccharide, Salze davon, Derivate davon, Konjugate davon und Mischungen davon.

10. Verfahren nach einem der vorhergehenden Ansprüche, **dadurch gekennzeichnet, dass** es sich bei der Komponente A um Albumin, insbesondere Serumalbumin, bevorzugt humanes Serumalbumin, handelt.

11. Verfahren nach einem der vorhergehenden Ansprüche, **dadurch gekennzeichnet, dass** es sich bei der Komponente B und/oder einer gegebenenfalls zusätzlich vorgesehenen Komponente C um eine thiolreaktive Verbindung handelt, insbesondere ausgewählt aus der Gruppe umfassend Maleimidverbindungen, Vinylsulfonverbindungen, Acrylatverbindungen, Alkylhalogenidverbindungen, Azirinverbindungen, Pyridinverbindungen, Thionitrobenzoesäureverbindungen, Arylverbindungen, Derivate davon und Mischungen davon.

12. Verfahren nach einem der vorhergehenden Ansprüche, **dadurch gekennzeichnet, dass** es sich bei der Komponente B und/oder einer gegebenenfalls zusätzlich vorgesehenen Komponente C um ein Maleimidderivat handelt.

13. Verfahren nach einem der vorhergehenden Ansprüche, **dadurch gekennzeichnet, dass** die Komponente B und/oder eine gegebenenfalls zusätzlich vorgesehene Komponente C ausgewählt sind aus der Gruppe umfassend 3-Maleimidopropionsäure, 3-Maleimidopropionsäure-N-Hydroxysuccinimidester und Kombinationen davon.

14. Produkt, vorzugsweise in Form einer funktionalisierten hochmolekularen Komponente wie beispielsweise eines funktionalisierten Proteins, hergestellt oder herstellbar nach einem Verfahren nach einem der vorhergehenden Ansprüche.

15. Verwendung eines Dialysators oder einer Dialysekassette zur Durchführung eines Verfahrens nach einem der Ansprüche 1 bis 13.

16. Verwendung einer Dialysevorrichtung zur Durchführung eines Verfahrens nach einem der Ansprüche 1 bis 13.
